# EUROPEAN PATENT APPLICATION

(11) **EP 2 241 310 A2**
(43) Date of publication of application: **20.10.2010**
(21) Application number: 10160217.5
(22) Date of filing: 16.04.2010
(51) Int. Cl.: A61K 9/20, A61K 31/44, A61K 9/28

(54) **Modified release formulations of emoxypine**

(30) Priority: 17.04.2009 TR 200903013; 28.05.2009 TR 200904159
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Türkyilmaz, Ali, 34398, Istanbul (TR)
(74) Representative: Sevinç, Erkan

(57) **Abstract**

The present invention is related to a modifed release pharmaceutical formulations comprising emoxypine or a pharmaceutically acceptable salt thereof and a rate controlling polymer, with at least one pharmaceutically acceptable excipient.

## Description

### Technical Aspect

The present invention is related to modifed release pharmaceutical formulations comprising emoxypine or a pharmaceutically acceptable salt thereof and a rate controlling polymer, with at least one pharmaceutically acceptable excipient.

### Background of the Invention

Emoxypine is a heteroaromatic antioxidant which has a membranoprotective, nootropic and sedative action. Its aromatic activity range is very wide such as increasing the stress resistance of organism. It acts as the anxiolytic which doesn't cause the sleepiness and myorelaxant effect; its nootropic properties allow to prevent and reduce learning and memory defects in patients of old age under pathogenic factors. One of the activities of emoxypine is its anticonvulsant action which shows antioxidant and antihypoxic properties. It increases the attention concentration and capacity for work and decreases the alcohol toxic affect. It inhibits peroxide oxidation of lipids, increases the superoxidoxidase activity and elevates the ratio of lipid-protein. Also, provides a higher dopamine concentration in brain.

Its chemical name is 2-ethyl-6-methyl-3-hydroxypyridine and its chemical structure is shown in the Formula I.

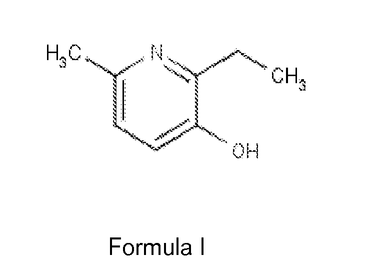

Emoxypine is marketed under the brand name MEXIDOL^{®} and it is administered orally in a therapeutic dose from 125 mg to 250 mg, three times a day and it has injectable formulations containing 50mg/ml in 2 ml and 5ml ampules in IV/IM forms.

In prior art, emoxypine is widely used as injectable preparations but it can not provide enough nootropic activity over a prolonged period in enjectable preparations. It has to be used in higher dosage ranges and longer time periods such as between 30 to 95 days which is not very suitable for the patient compliance and for the therapeutic activity ranges of emoxypine.

US patent no 4,486,440 in 17.08.1983, discloses injectable solutions and drops containing 1% of emoxypine HCl by weight. It is used as retinoprotector for treating intraocular hemorrhage, myopic chorioretinal dystrophies, congenital retinal dystrophies, retinal burns and prevention of injury in lasercoagulation.

A conventional tablet formulation of emoxypine succinate (Mexidol^{®}) is described in PCT application WO 96/02238 A1, Aktsionernoe Obschestvo Zakrytogo Tipa 'Olvik', 15.07.1994. The proposed neurotropic and sedative formulation contains emoxypine and auxiliary agents in core and a coating layer. The auxiliary agents in the core comprise kaolin, potato starch, calcium stearate, stearic acid, talc and methyl cellulose. The coating layer contains titanium dioxide and serge 80.

It is desirable in the treatment of a number of diseases, to provide the active pharmaceutical ingredient in a modified release form. Desirably the modified release provides a generally uniform and constant rate of release over a modified period of time which achieves desired plasma level of the active ingredient without the need for frequent administration of the medicament.

Accordingly, a need rises for modified release formulations of emoxypine or pharmaceutically acceptable salt, which overcomes the above described problems and minimizes the adverse effects and provides a comperable drug plasma concentration which is equivalent to or better than the immediate release tablet or injectable formulations currently used. The formulation of this invention represents a novel modified release formulation previously undisclosed in the prior art.

The modified relase formulation of this invention further provides the advantageous property of allowing the active medicament to be administered less frequently, e.g. once-a-day or twice-a-day, while achieving comparable plasma levels to immediate release forms.

### Description of the invention

The main embodiment of the present invention is to provide a novel modified release formulation comprising emoxypine or a pharmaceutically acceptable salt thereof and a rate controlling polymer, with at least one pharmaceutically acceptable excipient which overcomes the above described problems in prior art and have an additive advantages over them.

"Modified release dosage forms" are defined as those whose drug release characteristics of time course and/or location are chosen to accomplish therapeutic or convenience objectives not offered by conventional forms. A modified release dosage form potentially provides greater effectiveness in the treatment of chronic conditions; greater convenience; reduces side effects and provides higher levels of patient compliance or therapeutic performance due to a simplified dosage schedule, compared with those of immediate-release drugs. Modified release pharmaceutical products are formulated to release the drug's active ingredient gradually and predictably over a 12-hour to 24-hour period.

The USP (United States Pharmacopeia) considers that the terms controlled release; prolonged release extended release and sustained release is interchangeable with modified release. Accordingly, the terms "modified-release", controlled-release", "prolonged-release", "extended-release", and "sustained-release" are used interchangeably herein.

One of the advantages of modified release formulation of this invention for the outpatient is reduced dosage regimens convenience and, more importantly, better assurance of compliance. For example, the reduction of a dose regimen from four times a day to three times a day allows the patient to take the prescribed drug during waking hours. Reduction of a dose regimen to twice a day allows the patient to take the prescribed drug in the morning and in the evening, which provides greater convenience; e.g., the patient is not required to carry an additional one when away from home. Of course, the most convenient dosage form is a once daily dose regimen. This also reduces the risk of the omitted tablets.

The other advantage of modified release formulations is to make the plasma concentration level stable by maintaining the drug in the blood stream for a longer time period. In this manner, the formulation of this invention is designed as modified release to prevent the changes in drug plasma concentration levels and can be administered only once or twice a day for drugs that would otherwise have to be taken more frequently to maintain required blood levels. Also modified release oral dosage forms may help to treat with reduced dosage regimens. Using lower dosages of active ingredients provide greater convenience and reduced side effects and toxicity.

Another advantage of this modified release formulations of this invention is to prevent dose dumping. Dose dumping is one of the most important disadvantages of modified release dosage forms. Because of several different reasons it is difficult to develop modified release formulations of highly soluble pharmaceuticals although there are many modified release formulations formulated with rate controlling polymers. First of all, modified release formulations of highly soluble medicaments can be prone to "dose dumping" in which the release of the active ingredient is delayed but once the release begins the medicament is released very fast. The most important criteria of dose dumping under in-vitro conditions, is the amount of the active substance released in early time point.

It is also hard to achieve the desired dissolution profiles in other words the control of the release rate is difficult. Therefore, fluctuation of the active ingredient concentration in the plasma may occur which may lead to toxicity. Also, diurnal variation of the active ingredient in plasma is also possible.

As used here in, 'rate controlling polymer' means an excipient in the final dosage form whose primary function is to modify the duration of release of the active drug substance from the dosage form.

In one of the embodiments, the amount of the rate controlling polymer in the formulation generally varies from about 1.0% to 80.0% by weight of the formulation. Preferably, the amount of rate controlling polymer is from about 5.0% to about 70.0% by weight of the formulation. Most preferably, the amont of the rate controlling polymer is from about 10.0% to about 60.0% by weight of the formulation.

According to another embodiment of this invention, the modified release formulation comprises emoxypine or a pharmaceutically acceptable salt in an amount of about 1.0% to 80.0% by weight of total formulation, preferably about 20.0% to 75.0% by wieght, the most preferably about 45.0% to 70.0% by weight. Emoxypine is preferably in the form of succinate or HCl salt.

In a further aspect, the present invention relates to the modified release formulations comprising emoxypine or a pharmaceutically acceptable salt thereof, in an amount of 1 mg to 1000mg. Preferably 50 mg to 800 mg, the most preferably 125 mg to 750 mg.

The modified release formulation of this present invention is administered in the form of a once-a-day or twice-a-day dosage regimen.

It has surprisingly found that in this modified release formulation having a weight ratio of emoxypine or a pharmaceutically acceptable salt to rate controlling polymer between the ranges of about 1:10 to 10:1 (w/w) has a synergistic effect over the dissolution rate.

Another advantage of this invention is to provide a modified release formulation with a rate controlling polymer, wherein the maximum 30% of total amount of emoxypine or a pharmaceutically acceptable salt is released in 2 hours and 40-65% in 6 hours and at least 85% between in a period of 16 to 20 hours.

Rate controlling polymers can be used to form a matrix in which an active substance is dispersed, the properties of the polymer are then utilised control the rate at which the active is released from the formulation. The rate controlling polymers of this invention, are selected from the group comprising cellulosic polymers, polyvinylpyrrolidine, polyethylene oxide, glyceryl behenate, vinyl acetate/crotonic acid copolymers, maleic anhydride/methyl vinyl ether copolymers, copolymer of acrylic and methacrylic acid esters, shellac, waxes, zein and the like and mixtures thereof and alginate salts and complex salt of alginic acid.

According to this invention, the preferred cellulosic polymers are selected from the group comprising hydroxypropylmethyl cellulose, hydroxypropyl cellulose, low-substituted hydroxylpropyl cellulose, hydroxyethyl cellulose, ethyl cellulose, methyl cellulose, sodium carboxy methyl cellulose, cellulose acetatebutyrate and the like and mixtures thereof.

The cellulosic polymer is preferably hydroxypropyl methylcellulose. The amount of hydroxypropyl methylcellulose is present about 1.0 to 80.0% by weight of total formulation, particularly about 10.0% to 60.0%, more particularly about 20.0% to 50.0% by weight.

The amount of rate controlling polymer, hydroxypropyl methylcellulose is preferably adjusted to provide a release of maximum 30% of the total amount of emoxypine or a pharmaceutically acceptable salt in 2 hours and 40-65% in 6 hours and at least 85% between in a period of 16 to 20 hours.

The cellulosic polymer is preferably ethyl cellulose. The amount of ethyl cellulose is present about 1.0 to 40.0 % by weight of total formulation, particularly about 1.0 to 20.0%.

The amount of rate controlling polymer, ethyl cellulose is preferably selected to provide a release of maximum 30% of the total amount of emoxypine or a pharmaceutically acceptable salt in 2 hours and 40-65% in 6 hours and at least 85% between in a period of 16 to 20 hours.

The rate controlling polymer is preferably alginate salt which is selected from the group comprising sodium alginate, calcium alginate, potassium alginate, propylene glycol alginate, and the like and mixtures thereof.

The alginate salt is preferably sodium alginate. The amount of sodium alginate is about 1.0 to 20.0% by weight of total formulation.

The amount of rate controlling polymer, sodium alginate is preferably selected to provide a release of maximum 30% of the total amount of emoxypine or a pharmaceutically acceptable salt in 2 hours and 40-65% in 6 hours and at least 85% between in a period of 16 to 20 hours.

The rate controlling polymer is preferably the complex salt of alginic acid which is sodium-calcium alginate. The amount of sodium-calcium alginate is about 1.0 to 10.0% by weight of total formulation.

The amount of rate controlling polymer, sodium-calcium alginate is preferably selected to provide a release of maximum 30% of the total amount of emoxypine or a pharmaceutically acceptable salt in 2 hours and 40-65% in 6 hours and at least 85% between in a period of 16 to 20 hours.

The modified release formulations of this invention further comprising at least one pharmaceutically acceptable excipient selected from the group comprising diluents, fillers, disintegrants, binders, lubricants, glidants and preservatives.

Suitable diluents and fillers may include but not limited to lactose, microcrystalline cellulose, starch, talc, mannitol, glucose, and the like and mixtures thereof; preferably lactose and starch.

Suitable disintegrants may include but not limited to microcrystalline cellulose, sodium starch glycollate, croscarmellose sodium, crospovidone, starch and the like and mixtures thereof; preferably microcrystalline cellulose and croscarmellose sodium.

Suitable binders may include but not limited to polyvinylpyrrolidone, sucrose, polyethylene glycol and the like and mixtures thereof; preferably polyvinylpyrrolidone.

Suitable lubricants may include but not limited to sodium stearyl fumarate, stearic acid, magnesium strearate, calcium stearate and the like and mixtures thereof; preferably sodium stearyl fumarate, magnesium stearate and stearic acid.

Suitable glidants may include but not limited to colloidal silicon dioxide, talc, aluminium silicate and the like and mixtures thereof; preferably colloidal silicon dioxide.

Suitable preservatives may include but not limited to methyl paraben, propyl paraben, sodium benzoate, citric acid and benzoic acid and the like and mixtures thereof; preferably citric acid.

These modified release pharmaceutical formulations of this invention, are administrated by oral, parenteral, intramuscular or topical route. The modified release pharmaceutical formulations of this invention include tablet, capsule, sachet, microcapsules, minitablet, multilayer and multicoated tablet, pellet, injectable preparation, suspension, syrup, gel, cream or ointment which can be formulated in accordance with methods that are standard in the art. It is preferably in the form of tablets or capsules.

The formulations of the present invention may be prepared by conventional technology well known to those skilled in the art such as wet granulation, direct compression, dry granulation and the like. Thus, for instance, emoxypine, or a pharmaceutically acceptable salt thereof, a rate controlling polymer and other excipients are mixed together to form the modified release formulations. The prepared mixture is filled into capsules or compressed to form tablets.

In another embodiment, the modified relase pharmaceutical dosage forms may comprise a coating layer. The coating layer preferably comprises, for example, zein, hydroxyproply cellulose, hydroxypropyl methylcellulose, ethly cellulose, triacetin, dibutyl sebacate, lactose, talc, polyethylene glycol, polyvinyl alcohol, polysorbate, titanium dioxide, iron oxide, Food and Drug Cosmetic (FD&C) dyes (such as; FD&C blue, FD&C green, FD&C red, FD&C yellow, FD&C lakes) and the like and mixtures thereof.

According to another embodiment of the modified relase formulations of this invention, core or coating may comprise enteric coating.

The enteric coating layer may comprise cellulose acetate phthalate, methyl acrylate-methacrylic acid copolymers, cellulose acetate succinate, shellac, hydroxypropyl methylcellulose phthalate, hydroxypropyl methylcellulose acetate, hypromellose acetate succinate, polyvinyl acetate phthalate, cellulose acetate phthalate, polymethacrylates, methyl methacrylate-methacrylic acid copolymers and the like and mixtures thereof.

Another preferred embodiment of this invention is a modified release formulation which is in the form of a tablet comprising:
(a) a core comprising the emoxypine or a pharmaceutically acceptable salt thereof and pharmaceutically acceptable excipients,
(b) a coating layer comprising the rate controlling polymer which provides slow release.

The manufacturing process is preferably performed by mixing the components, wet granulating the mixed components; the mixed components, drying the mixture, milling the dried mixture, blending the mixture with a lubricant(s) and compressing the blended mixture to form tablets or filling the blended mixture into capsules.

A preferred process for preparing the modified release formulations of the invention comprises the following steps;
(a) mixing emoxypine or a pharmaceutically acceptable salt thereof and a rate controlling polymer and other excipients;
(b) wet granulating the mixed components;
(c) drying the mixture;
(d) milling the dried mixture;
(e) blending the mixture with a lubricant; and
(f) compressing the blended mixture to form tablets, and optionally coating said tablets.

Another embodiment of the present invention is to provide an orally administrable modified release pharmaceutical dosage form comprising emoxypine and a rate controlling polymer with at least one pharmaceutically acceptable excipient wich has a wide spectrum of pharmacological activities such as membranoprotection, neuroprotection, cardioprotection, antihypoxic, anxiolytic, nootropic, antiamnestic, antiatherogenic, antistress and antialcoholic actions.

Another action of emoxypine is seen on neuro-and the effective status in patients at early stages of diabetic foot syndrome when it is used in combination with alpha-lipoic acid. Emoxypine also improves learning and memory in hemorrhagic stroke and exerted influence on the locomotor activity.

It is also reported that emoxypine can be administered in combination with non-narcotic analgesics, in particular with Pentalgin, for relieving painful syndrome on the background of stress. Also it may be effective in combination with valproic acid (sodium valproate-Depakine^{®}) for treatment of patients with generalized epilepsy and as monotherapy in patients with first episode of febrile seizures.

Another action of emoxypine is eliminating anxiety and fear, recovering adequate reactions and the orientative-trying behavior, and lessens aggressiveness.

This invention is further defined by reference to the following examples. Although the example is not intended to limit the scope of the present invention, it should be considered in the light of the description detailed above. It will be apparent to those skilled in the art that many modifications, both to materials and methods, may be practiced without departing from the scope of the invention.

### Example 1

The modified release formulation, comprises about 1.0 to 80.0% by weight of emoxypine or a pharmaceutically acceptable salt, about 1.0 to 80.0% by weight HPMC (hydroxypropyl methylcellulose), about 5.0 to 60.0% by weight of lactose, about 0.50 to 20.0% by weight of polyvinylpyrrolidone, about 5.0 to 60.0% by weight of microcrystalline cellulose, about 0.10 to 5.0% by weight of sodium stearyl fumarate, about 0.10 to 5.0% by weight of colloidal silicon dioxide.

### Example 2

The modified release formulation, comprises about 1.0 to 80.0 % by weight of emoxypine or a pharmaceutically acceptable salt, about 1.0 to 40.0 % by weight ethyl cellulose, about 5.0 to 60.0% by weight of starch, about 0.50 to 20.0 % by weight of polyvinylpyrrolidone, about 5.0 to 60.0 % by weight of crosscarmellose sodium, about 0.10 to 5.0 % by weight of sodium stearyl fumarate, about 0.10 to 5.0 % by weight of colloidal silicon dioxide.

### Example 3

The modified release formulation, comprises about 1.0 to 80.0 % by weight of emoxypine or a pharmaceutically acceptable salt, about 1.0.to 20.0 % by weight sodium alginate, about 5.0 to 60.0 % by weight of lactose about 0.50 to 20.0 % by weight of polyvinylpyrrolidone, about 5.0 to 60.0 % by weight of microcrystalline cellulose, about 0.10 to 5.0 % by weight of magnesium stearate, about 0.10 to 5.0 % by weight of colloidal silicon dioxide and about 5.0 to 30.0 % by weight of citric acid.

The formulations of these examples are manufactured according to the process described above in the description and was tested for its dissolution profile using a USP paddle method. These tablet formulations may comprise a coating layer and this coating layer may comprise enteric coating.

## Claims

1. A modified release pharmaceutical formulation comprising emoxypine or a pharmaceutically acceptable salt thereof and a rate controlling polymer which is selected from the group comprising hydroxypropylmethyl cellulose, low-substituted hydroxylpropyl cellulose, ethyl cellulose, methyl cellulose, cellulose acetatebutyrate, polyethylene oxide, glyceryl behenate, vinyl acetate/crotonic acid copolymers, maleic anhydride/methyl vinyl ether copolymers, copolymer of acrylic and methacrylic acid esters, shellac, waxes, zein, alginate salts and complex salt of alginic acid and the like and mixtures thereof with at least one pharmaceutically acceptable excipient.

2. The modified release pharmaceutical formulation according to claim 1, wherein the amount of the rate controlling polymer is from 1.0% to 80.0% by weight, preferabaly it is from 5.0 to 70.0% by weight, more preferably it is 10.0 to 60.0 % by weight of the formulation.

3. The modified release pharmaceutical formulation according to claim 1, wherein the rate controlling polymer is hydroxypropyl methylcellulose.

4. The modified release pharmaceutical formulation according to claim 1, wherein the rate controlling polymer is ethyl cellulose.

5. The modified release pharmaceutical formulation according to claim 1, wherein the rate controlling polymer is alginate salts comprising sodium alginate, calcium alginate, potassium alginate, propylene glycol alginate and the like and mixtures thereof, preferably the alginate salt is sodium alginate.

6. The modified release pharmaceutical formulation according to claim 1, wherein the rate controlling polymer is complex salt of alginic acid, preferably the complex salt of alginic aicd is sodium-calcium alginate.

7. The modified release formulation according to claim 6, wherein the amount of sodium-calcium alginate is from 1.0 to 10.0% by weight of total formulation.

8. The modified release formulation according to any preceding claims, wherein the weight ratio of emoxypine to rate controlling polymer is about 1:10 to 10:1.

9. The modified release formulation according to claims 1 to 8, wherein emoxypine or a pharmaceutically acceptable salt is present in an amount of 1.0 to 80.0% by weight, preferably it is 20.0 to 75.0% by weight, more preferably it is 45.0 to 70.0% by weight.

10. The modified release formulation according to claims 1 to 9, wherein emoxypine or a pharmaceutically acceptable salt is in an amount of 1 mg to 1000 mg.

11. The modified release formulation according to claims 1 to 10, wherein the formulation is in the form of a once-a-day or twice-a-day dosage regimen.

12. The modified release formulation according to claims 1 to 11, wherein the maximum 30% of total amount of emoxypine or a pharmaceutically acceptable salt is released in 2 hours and 40-65% in 6 hours and at least 85% between in a period of 16 to 20 hours.

13. The modified release formulation according to any preceding claims, further comprising at least one pharmaceutically acceptable excipient selected from the group comprising diluents, fillers, disintegrants, binders, lubricants, glidants and preservatives.

14. The modified release formulation according to any preceding claims, comprising about 1.0 to 80.0% by weight of emoxypine or a pharmaceutically acceptable salt, about 1.0 to 80.0% by weight HPMC (hydroxypropyl methylcellulose), about 5.0 to 60.0% by weight of lactose, about 0.50 to 20.0% by weight of polyvinylpyrrolidone, about 5.0 to 60.0% by weight of microcrystalline cellulose, about 0.10 to 5.0% by weight of sodium stearyl fumarate, about 0.10 to 5.0% by weight of colloidal silicon dioxide.

15. The modified release formulation according to any preceding claims, comprising about 1.0 to 80.0% by weight of emoxypine or a pharmaceutically acceptable salt, about 1.0 to 40.0% by weight ethyl cellulose, about 5.0 to 60.0% by weight of starch, about 0.50 to 20.0% by weight of polyvinylpyrrolidone, about 5.0 to 60.0% by weight of crosscarmellose sodium, about 0.10 to 5.0% by weight of sodium stearyl fumarate, about 0.10 to 5.0% by weight of colloidal silicon dioxide

16. The modified release formulation according to any preceding claims, comprising about 1.0 to 80.0% by weight of emoxypine or a pharmaceutically acceptable salt, about 1.0 to 20.0% by weight sodium alginate, about 5.0 to 60.0% by weight of lactose about 0.50 to 20.0% by weight of polyvinylpyrrolidone, about 5.0 to 60.0 % by weight of microcrystalline cellulose, about 0.10 to 5.0% by weight of magnesium stearate, about 0.10 to 5.0 % by weight of colloidal silicon dioxide and about 5.0 to 30.0 % by weight of citric acid.

17. The modified release formulation according to claims 1 to 16, wherein emoxypine is in the form of a succinate salt.

18. The modified release formulation according to claims 1 to 16, wherein emoxypine is in the form of a HCl salt.

19. The modified release formulation according to any preceding claims, further comprising a coating layer.

20. The modified release formulation according to claim 19, wherein the coating layer comprising zein, hydroxyproply cellulose, hydroxypropyl methylcellulose, ethly cellulose, triacetin, dibutyl sebacate, lactose, talc, polyethylene glycol, polyvinyl alcohol, polysorbate, titanium dioxide, iron oxide, Food and Drug Cosmetic (FD&C) dyes and the like and mixtures thereof.

21. The modified release formulation according to claim 19, wherein the coating layer is preferably enteric coating.

22. The modified release formulation according to claim 21, wherein the enteric coating layer comprising cellulose acetate phthalate, methyl acrylate-methacrylic acid copolymers, cellulose acetate succinate, shellac, hydroxypropyl methylcellulose phthalate, hydroxypropyl methylcellulose acetate, hypromellose acetate succinate, polyvinyl acetate phthalate, cellulose acetate phthalate, polymethacrylates, methyl methacrylate-methacrylic acid copolymers and the like and mixtures thereof.

23. The modified release formulation according to claims 19 to 22, in the form of a tablet comprising:
(a) a core comprising the emoxypine or a pharmaceutically acceptable salt thereof and pharmaceutically acceptable excipients,
(b) a coating layer comprising the rate controlling polymer which provides slow release.

24. The modified release formulation according to any preceding claim, wherein the formulation is to be administrated by orally.

25. The modified release formulation according to any preceding claim, wherein the formulaiton is in the form of a tablet, capsule, sachet, microcapsule, minitablet, pellet, multilayer and multicoated tablet.

26. A process for preparing modified release formulations according to claims 1 to 22 and 24 to 25 which comprises;
(a) mixing emoxypine or a pharmaceutically acceptable salt thereof and a rate controlling polymer and other excipients;
(b) wet granulating the mixed components;
(c) drying the mixture;
(d) milling the dried mixture;
(e) blending the mixture with a lubricant; and
(f) compressing the blended mixture to form tablets, and optionally coating said tablets.
